# EUROPEAN PATENT SPECIFICATION

(11) **EP 1 674 450 B1**
(45) Date of publication and mention of the grant of the patent: **10.11.2010**
(21) Application number: 05027960.3
(22) Date of filing: 20.12.2005
(51) Int. Cl.: C07C 249/04, C07C 251/44

(54) **Process for producing cyclohexanone oxime**
Verfahren zur Herstellung von Cyclohexanonoxim
Procédé de préparation de l'oxime de la cyclohexanone

(30) Priority: 22.12.2004 JP 2004370725
(43) Date of publication of application: 28.06.2006
(73) Proprietor: Sumitomo Chemical Company, Limited, Tokyo 104-8260 (JP)
(72) Inventor: Trivellone, Franco, 20052 Monza (IT); Furlan, Piero, 31100 Treviso (IT); Oikawa, Miyuki, Niihama-shi Ehime-ken (JP)
(74) Representative: Vossius & Partner

(56) References cited:
- EP-A- 0 496 385
- EP-A- 1 468 986

## Description

The present invention relates to a process for producing cyclohexanone oxime by an ammoximation reaction of cyclohexanone. Cyclohexanone oxime is useful as, for example, the raw material of ε-caprolactam.

**As one of the** processes for producing cyclohexanone oxime, there is a proposed process by carrying out the ammoximation of cyclohexanone with hydrogen peroxide and ammonia in the presence of a titanosilicate catalyst (see, for example, Japanese Patent Kokai Publications No. S62-59256, No. H6-49015, No. H6-92922 and No. 2000-72737). This process has advantages such as no need of neutralization of sulfuric acid with ammonia, which had been necessary for a conventional process to carry out oximation with hydroxylamine sulfate, and easy separation of the product from the catalyst since the catalyst used for the reaction is a solid catalyst.

According to the above-mentioned ammoximation reaction, however, the titanosilicate catalyst is gradually deteriorated as the reaction time passes, and this may cause a lowered conversion ratio of cyclohexanone and/or a lowered selectivity of cyclohexanone oxime. Therefore, Japanese Patent Kokai Publication No. H6-92922 discloses that on carrying out the ammoximation reaction in a continuous manner by supplying cyclohexanone, hydrogen peroxide and ammonia to a rector while taking out through a filter a liquid phase of the reaction mixture (in which the catalyst was dispersed) from the reactor, the catalyst which is deteriorated is discontinuously taken out and replaced by a fresh catalyst to maintain the catalyst activity. Further, Japanese Patent Kokai Publication No. 2000-72737 discloses that the ammoximation reaction is carried out in a continuous manner while supplying the catalyst together with the raw materials to the reactor.

If such replacement or introduction of the catalyst as described above is frequently carried out, this is advantageous for maintaining the conversion ratio of cyclohexanone and the selectivity of cyclohexanone oxime, but disadvantageous in view of cost because the amount of the catalyst used is increased. Accordingly, the purpose of the present invention is to provide a process which is able to suppress the catalyst cost as low as possible and maintain the conversion ratio of cyclohexanone and the selectivity of cyclohexanone oxime in the ammoximation reaction of cyclohexanone so as to produce cyclohexanone oxime with good productivity over a long period.

The present invention provides a process for producing cyclohexanone oxime by continuously reacting cyclohexanone, hydrogen peroxide and ammonia in the presence of a titanosilicate catalyst and optionally a solvent, wherein the process comprises the first operation step in which the reaction is carried out while raising the concentration of the catalyst in the reaction mixture by adding catalyst to the reaction mixture continuously or intermittently; and the second operation step in which the reaction is carried out keeping the concentration of the catalyst in the reaction mixture in as predetermined range by taking out catalyst from the reaction mixture and adding catalyst to the reaction mixture.

According to the present invention, in the continuous ammoximation reaction of cyclohexanone, the catalyst cost is suppressed as low as possible; and the conversion ratio of cyclohexanone and the selectivity of cyclohexanone oxime can be maintained, and thus cyclohexanone oxime can be produced with good productivity over a long period.

The present invention is explained in more detail below. Titanosilicate used for the present invention is a zeolite(s) which contains titanium, silicon, and oxygen as elements composing its framework. The zeolite may have the framework substantially composed of titanium, silicon, and oxygen, or the zeolite may further coritain other element(s) as elements composing the framework. As to titanosilicate, those having an atomic ratio of silicon/titanium from 10 to 1000 are preferably used, and it may be in the form of, for example, fine powder or pellets. Titanosilicate can be prepared by, for example, a process described in Japanese Patent Kokai Publication No. S56-96720.

Cyclohexanone oxime can be produced by using titanosilicate described above as the catalyst and carrying out the ammoximation reaction of cyclohexanone with hydrogen peroxide and ammonia in the presence of this catalyst.

Cyclohexanone as the raw material may be, for example, that obtained by an oxidation reaction of cyclohexane, that obtained by hydration and dehydrogenation reactions of cyclohexene, or that obtained by a hydrogenation reaction of phenol.

Hydrogen peroxide is usually produced by a so-called anthraquinone process. It is commercially available in the form of an aqueous solution which generally has a concentration from 10 to 70% by weight, and such aqueous solution of hydrogen peroxide can be used for the present invention. The amount of hydrogen peroxide used is preferably 0.5 to 3 moles, and more preferably 0.5 to 1.5 moles per 1 mole of cyclohexanone. It is noted that stabilizers of, for example, a salt of phosphoric acid such as sodium phosphate, a salt of polyphosphoric acid such as sodium pyrophosphate and sodium tripolyphosphate, pyrophosphoric acid, ascorbic acid, ethylenediaminetetraacetic acid, nitrotriacetic acid, aminotriacetic acid, and diethylenetriaminepentaacetic acid may be added to hydrogen peroxide.

As to ammonia, gaseous ammonia may be used, or liquid ammonia may be used, and it may be in the form of a solution in water or an organic solvent. The amount of ammonia used is preferably 1 mole or more, and more preferably 1.5 moles or more per 1 mole of cyclohexanone.

The above-mentioned ammoximation reaction may be carried out in a reaction solvent. As the reaction solvent, an.alcohol such as methyl alcohol, ethyl alcohol, n-propyl alcohol, isopropyl alcohol, n-butyl alcohol, sec-butyl alcohol, tert-butyl alcohol or tert-amyl alcohol, water, or a mixed solvent thereof is preferably used.

The reaction temperature of the above-mentioned ammoximation reaction is usually 50°C to 120°C, and preferably 70°C to 100°C. The reaction pressure may be a normal pressure (or an atmospheric pressure), but the reaction is preferably carried out under an increased pressure to increase the amount of ammonia solved in the liquid phase of the reaction mixture, and in this case, the pressure may be adjusted by using an inactive gas (es) such as nitrogen, helium or the like.

In the present invention, the above-mentioned ammoximation reaction is carried out in a continuous manner. Such continuous reaction is usually conducted by retaining a certain amount of the reaction mixture (in which a catalyst is dispersed) in a continuous-type reactor such as a stirred vessel and a loop-type vessel, and supplying cyclohexanone, hydrogen peroxide, ammonia, and a solvent or the like if necessary, to the reactor, while taking out the reaction mixture at a rate nearly equal to that of these raw materials supplied to the reactor. At the start of the reaction, for example, a catalyst-dispersed solution where the catalyst is dispersed in a solvent is charged in the reactor, and then the supply of the raw materials thereto is started, and the taking out of the reaction mixture therefrom is also started. The concentration of the catalyst in the catalyst-dispersed solution as the charged solution, i.e. the initial concentration of the catalyst, is usually 1 to 50 g/L, and preferably 5 to 40 g/L, and more preferably 10 to 30g/L as a weight per unit volume of the charged solution (catalyst + liquid phase), though it depends on the activity of the catalyst, reaction conditions and so on. It is noted that the reactor is preferably a glass-lined one or made of stainless steel from the viewpoint of prevention of the decomposition of hydrogen peroxide.

In the present invention, the continuous ammoximation reaction is composed of the first operation step in which the reaction is carried out while raising the concentration of the catalyst in the reaction mixture by adding the catalyst to the reaction system, and the second operation step in which the reaction is carried out while keeping the concentration of the catalyst in the reaction mixture in a predetermined range by taking out the catalyst from the reaction system and adding a catalyst to the reaction system. By applying such multistep operating condition for the addition and taking out of the catalyst as described above, it becomes possible to maintain the activity of the catalyst and the reaction selectivity, i.e. the conversion ratio of cyclohexanone and the selectivity of cyclohexanone oxime advantageously also in view of cost, and thus to produce cyclohexanone oxime with good productivity over a long period. It should be noted that as the catalyst added, a fresh catalyst which has not been used for the reaction is usually used, but a recovered catalyst which has been already used for the reaction can be recycled after being subjected to regeneration treatment if necessary, or the fresh catalyst and the recovered catalyst may be used in combination with each other.

In the first operation step, the addition of the catalyst may be carried out continuously or intermittently. On the other hand, the taking out of the catalyst is not basically carried out excluding a little portion which is dissolved into the liquid phase of the reaction mixture. Accordingly, the catalyst is accumulated to raise the concentration of the catalyst. For this purpose, it is better to take out the reaction mixture by taking out only the liquid phase through a filter or the like. It is possible by accumulating the catalyst to raise its concentration as described above to reduce the amount of the catalyst added which is required for maintaining the conversion ratio or the selectivity at a certain level. It is noted that the concentration of the catalyst in the reaction mixture can be raised continuously when the addition of the catalyst is carried out continuously, and the concentration of the catalyst in the reaction mixture can be raised intermittently when the addition of the catalyst is carried out intermittently.

As the above-described first operation step is continued longer, it becomes harder to keep a good mixing condition where the catalyst is homogeneously dispersed because of the raising in the concentration of the catalyst in the reaction mixture. Further, a side reaction caused by accumulation of the deteriorated catalyst may become a problem. Therefore, when the concentration of the catalyst in the reaction mixture reaches a predetermined concentration in the first operation step, the operation is switched to the second operation step in which the reaction is carried out while keeping the concentration of the catalyst in a predetermined range by the taking out and the addition of the catalyst.

In the second operation step, each of the taking out and the addition of the catalyst may be carried out continuously or intermittently. In a case where the taking out and the addition of the catalyst is carried out intermittently, both of them may be carried out at the same time or at different timings. The concentration of the catalyst in the reaction mixture during the second operation step, i.e. the steady concentration of the catalyst, is usually 5 to 100 g/L, and preferably 20 to 100 g/L, and more preferably 60 to 100 g/L as a weight per unit volume of the reaction mixture (catalyst + liquid phase), though it depends on the activity of the catalyst, reaction conditions and so on.

The timing and the amount of the addition of the catalyst in the first operation step and the taking out and the addition of the catalyst in the second operation step are preferably adjusted depending on a concentration of the components in the reaction mixture. For example, since the residual concentration of cyclohexanone is a measure for the deterioration degree of the catalyst, it is better to carry out the taking out or the addition of the catalyst so as to keep the residual concentration not larger than a predetermined value, specifically, so as to keep it as a concentration of cyclohexanone in the liquid phase of the reaction mixture taken out from the reaction system not larger than 2% by weight, and preferably not larger than 1% by weight, and more preferably not larger than 0.5% by weight. Since the residual concentration of hydrogen peroxide is also a measure for the deterioration degree of the catalyst, it is better to carry out the taking out or the addition of the catalyst so as to keep the residual concentration not larger than a predetermined value, specifically, so as to keep it as a concentration of hydrogen peroxide in the liquid phase of the reaction mixture taken out from the reaction system not larger than 0.5% by weight, and preferably not larger than 0.2% by weight, and more preferably not larger than 0.05% by weight. As to ammonia, it is preferable for the conversion ratio of cyclohexanone and the selectivity of cyclohexanone oxime to use excess ammonia compared with cyclohexanone and hydrogen peroxide enough to remains, specifically so as to keep the concentration of ammonia in the liquid phase of the reaction mixture taken out from the reaction system not smaller than 1% and not larger than 10% by weight, and more preferably not smaller than 1.5% and not larger than 5% by weight.

Further, as a measure for the deterioration degree of the catalyst, it is also effective to determine the amount of a by-product gas(es) such as oxygen. Specifically, it is better to determine the concentration of oxygen in a waste gas discharged from the reactor by introducing an inactive gas(es) such as nitrogen or helium, and carry out the taking out or the addition of the catalyst when a steep rise in the concentration of oxygen is observed or before it is observed.

For a post-treatment operation of the reaction mixture thus obtained, known methods can be used if it is suitable. As described in Japanese Patent Kokai Publication No. H6-92922, for example, cyclohexanone oxime can be separated by concentrating the liquid phase of the reaction mixture, and then extracting cyclohexanone oxime from thus resultant concentrate into an organic solvent, followed by again concentrating the extract.

### Example

An example of the present invention is shown below, but the present invention is not limited by this. In the example, analysis for cyclohexanone and cyclohexanone oxime was conducted by a gas chromatography, and based on its results the conversion ratio of cyclohexanone, the selectivity of cyclohexanone oxime and the yield of cyclohexanone oxime were calculated.

A catalyst composed of crystalline titanosilicate which has an atomic ratio of silicon/titanium of 36 and has an MFI structure (manufactured by Polimeri Europa, and the crystalline titanosilicate having an MFI structure is hereinafter also referred to as TS-1) was used in the example. In this context, MFI means one of structure codes for zeolites defined by International Zeolite Association (IZA). The catalyst used herein was in the form of spherical secondary particles consisting of TS-1 primary particles bound to each other. A size of the particles of the catalyst was determined by a dry weight method using a particle size distribution measuring equipment (available from SAKURAGIRIKAGAKUKIKAI Co.). According to the results, a weight average particle size (or diameter) was 31 µm, and particles with a size not larger than 20 µm made up 15% by weight of total, and particles with a size not smaller than 105 µm made up 1% by weight of total.

### Example 1

An autoclave having a volume of 1 liter was used as a reactor, and 3 g of the catalyst and 500 g of hydroscopic tert-butyl alcohol (water content of 13% by weight) were charged and stirred in it, and retained at a temperature of 85°C and a pressure of 0.25 MPa (gauge pressure). Then, a continuous reaction was started under conditions of the temperature of 85°C and the pressure of 0.25 MPa (gauge pressure) by starting to supply 67 g/hr of cyclohexanone, 280 g/hr of a solution of 8 to 9% by weight of ammonia in hydroscopic tert-butyl alcohol (water content of 12% by weight), and 45 g/hr of a 60% by weight of hydrogen peroxide water to the autoclave, and by starting to take out the liquid phase of the reaction mixture through a filter so as to keep the amount of the reaction mixture in the reactor at 500 ml. The reaction was continued under the same conditions as that at the start of the reaction. During the reaction, while a gas of helium is introduced to the reactor at a flow rate of 5 L/hr, a waste gas was discharged through a pressure-keeping valve, and the concentration of oxygen in the waste gas was constantly determined by an oxygen concentration meter. The liquid phase of the reaction mixture which was taken out at 3.5 hours from the start of the reaction was analyzed, and the results showed that the conversion ratio of cyclohexanone was 90.2%, the selectivity of cyclohexanone oxime was 95.4%, and the yield of cyclohexanone oxime was 86.0%.

After 4 hours from the start of the reaction, a steep rise in the concentration of oxygen in the waste gas was observed, and therefore 1 g of the catalyst was added. Furthermore, after 16 hours from the start of the reaction, a steep rise in the concentration of oxygen in the waste gas was observed, so that 1 g of the catalyst was added. Then, the liquid phase of the reaction mixture which was taken out at 39 hours from the start of the reaction was analyzed, and the results showed that the conversion ratio of cyclohexanone was 99.3%, the selectivity of cyclohexanone oxime was 99.7%, and the yield of cyclohexanone oxime was 99.0%.

After 57 hours from the start of the reaction, a steep rise in the concentration of oxygen in the waste gas was observed, and 1 g of the catalyst was added while 1 g of the catalyst was taken out. These taking out and addition of the catalyst was carried out by taking out a portion of the reaction mixture without passing it through the filter, recovering the catalyst by filtration from the portion, then removing the catalyst of 1 g (dry weight basis) from the recovered catalyst, and returning the residual catalyst together with 1 g of the additional catalyst to the reactor (hereinafter, the operation was carried out in the same manner). Then, the liquid phase of the reaction mixture which was taken out at 75 hours from the start of the reaction was analyzed, and the results showed that the conversion ratio of cyclohexanone was 99.3%, the selectivity of cyclohexanone oxime was 99.7%, and the yield of cyclohexanone oxime was 99.0%.

After 89 hours from the start of the reaction, a steep rise in the concentration of oxygen in the waste gas was observed, so that 1 g of the catalyst was added while 1 g of the catalyst was taken out. Furthermore, after 107 hours from the start of the reaction, a steep raise in the concentration of oxygen in the waste gas was observed, so that 1g of the catalyst was added while 1 g of the catalyst was taken out. Then, the liquid phase of the reaction mixture which was taken out at 108 hours from the start of the reaction was analyzed, and the results showed that the conversion ratio of cyclohexanone was 98.3%, the selectivity of cyclohexanone oxime was 99.6%, and the yield of cyclohexanone oxime was 97.9%. After 121 hours from the start of the reaction, a steep raise in the concentration of oxygen in the waste gas was observed, and at that time point the reaction was terminated.

The present application claims priority under the Paris Convention to Japanese Patent Application No. 2004-370725 filed on December 22, 2004, entitled "PROCESS FOR PRODUCING CYCLOHEXANONE OXIME."

## Claims

1. A process for producing cyclohexanone oxime by continuously redacting cyclohexanone, hydrogen peroxide and ammonia in the presence of a titanosilicate catalyst and optionally a solvent, wherein the process comprises:
a first operation step in which the reaction is carried out while raising the concentration of the catalyst in the reaction mixture by adding catalyst to the reaction mixture continuously or intermittently; and
a second operation step in which the reaction is carried out keeping the concentration of the catalyst in the reaction mixture in as predetermined range by taking out catalyst from the reaction mixture and adding catalyst to the reaction mixture.

2. A process according to Claim 1, wherein the concentration of the titanosilicate catalyst in the reaction mixture is kept in a' range' from 5 to 100 g/L in the second operation step.

3. A process according to Claim 1 or 2, wherein a concentration of cyclohexanone in a liquid phase of the reaction mixture is kept at 2% by weight or less in the first operation step and the second operation step.

4. A process according to: any one of Claims 1 to 3, wherein a concentration of hydrogen peroxide in a liquid phase of the reaction mixture is kept at 0.5% by weight or less in the first operation step and the second operation step.

5. A process according to any one of Claims 1 to 4, wherein a concentration of ammonia in a liquid phase of the reaction mixture is kept at 1% by weight or more in the first operation step and the second operation step.

## Patentansprüche

1. Ein Verfahren zur Herstellung von Cyclohexanonoxim durch kontinuierliche Umsetzung von Cyclohexanon, Wasserstoffperoxid und Ammoniak in der Gegenwart eines Titanosilikat-Katalysators und gegebenenfalls eines Lösungsmittels, wobei das Verfahren umfasst:
einen ersten Arbeitsschritt, in welchem die Umsetzung durchgeführt wird, während die Konzentration des Katalysators in der Reaktionsmischung durch Zugabe von Katalysator zu der Reaktionsmischung kontinuierlich oder intermittierend erhöht wird; und
einen zweiten Arbeitsschritt, in welchem die Umsetzung durchgeführt wird, während die Konzentration des Katalysators in der Reaktionsmischung in einem vorgegebenen Bereich, durch Entfernen von Katalysator aus der Reaktionsmischung und Zugabe von Katalysator zu der Reaktionsmischung, gehalten wird.

2. Ein Verfahren gemäß Anspruch 1, wobei die Konzentration des Titanosilikat-Katalysators in der Reaktionsmischung in einem Bereich von 5 bis 100 g/L im zweiten Arbeitsschritt gehalten wird.

3. Ein Verfahren gemäß Anspruch 1 oder 2, wobei eine Konzentration von Cyclohexanon in einer flüssigen Phase der Reaktionsmischung bei 2 Gew.-% oder weniger in dem ersten Arbeitsschritt und dem zweiten Arbeitsschritt gehalten wird.

4. Ein Verfahren gemäß einem der Ansprüche 1 bis 3, wobei eine Konzentration von Wasserstoffperoxid in einer flüssigen Phase der Reaktionsmischung bei 0,5 Gew.-% oder weniger in dem ersten Arbeitsschritt und dem zweiten Arbeitsschritt gehalten wird.

5. Ein Verfahren gemäß einem der Ansprüche 1 bis 4, wobei eine Konzentration von Ammoniak in einer flüssigen Phase der Reaktionsmischung bei 1 Gew.-% oder mehr in dem ersten Arbeitsschritt und dem zweiten Arbeitsschritt gehalten wird.

## Revendications

1. Procédé pour la production d'oxime de cyclohexanone en faisant réagir en continu de la cyclohexanone, du peroxyde d'hydrogène et de l'ammoniac en présence d'un catalyseur à titanosilicate et, en option, d'un solvant, le procédé comprenant:
une première opération dans laquelle la réaction est exécutée en augmentant, de manière continue ou intermittente, la concentration du catalyseur dans le mélange réactionnel par addition du catalyseur au mélange réactionnel; et
une deuxième opération dans laquelle la réaction est exécutée en maintenant la concentration du catalyseur dans le mélange réactionnel dans un domaine prédéterminé en enlevant le catalyseur du mélange réactionnel et en ajoutant le catalyseur au mélange réactionnel.

2. Procédé selon la revendication 1, dans lequel la concentration du catalyseur à titanosilicate dans le mélange réactionnel est maintenue dans un domaine de 5 à 100 g/l au cours de la deuxième étape de l'opération.

3. Procédé selon la revendication 1 ou 2, dans lequel une concentration de' cyclohexanone dans une phase liquide du mélange réactionnel est maintenue à 2% en poids ou moins au cours de la première étape de l'opération et de la deuxième étape de l'opération.

4. Procédé selon l'une quelconque des revendications 1 à 3, dans lequel une concentration de peroxyde d'hydrogène dans une phase liquide du mélange réactionnel est maintenue à 0,5% en poids ou moins au cours de la première étape de l'opération et de la deuxième étape de l'opération.

5. Procédé selon l'une quelconque des revendications 1 à 4, dans lequel une concentration d'ammoniac dans une phase liquide du mélange réactionnel est maintenue à 1% en poids ou plus au cours de la première étape de l'opération et de la deuxième étape de l'opération.
